# EUROPÄISCHE PATENTSCHRIFT

(11) **EP 0 655 249 B1**
(45) Veröffentlichungstag und Bekanntmachung des Hinweises auf die Patenterteilung: **07.03.2001**
(21) Anmeldenummer: 94118415.2
(22) Anmeldetag: 23.11.1994
(51) Int. Cl.: A61K 31/70

(54) **Moenomycin als Arzneimittel zur Behandlung von Magengeschwüren**
Moenomycin as a medicament for the treatment of stomach ulcer
Moenomycin comme médicament pour le traitement des ulcères de l'estomac

(30) Priorität: 30.11.1993 DE 4340774
(43) Veröffentlichungstag der Anmeldung: 31.05.1995
(73) Patentinhaber: HOECHST AKTIENGESELLSCHAFT, 65929 Frankfurt am Main (DE)
(72) Erfinder: Riess, Dr. Günter, D-65795 Hattersheim (DE); Seibert, Prof. Dr. Gerhard, D-64291 Darmstadt (DE); Hedtmann, Dr. Udo, D-60433 Frankfurt (DE)

(56) Entgegenhaltungen:
- J.GEN.MICROBIOL., Bd.133, Nr.3, März 1987 Seiten 667 - 674 Y.VAN HEIJENOORT ET AL. 'Effects of Moenomycin on Escherichia coli'
- J.ANTIBIOT., Bd.30, Nr.12, Dezember 1977 Seiten 1060 - 1063 A.TORIKATA ET AL. 'PHOLIPOMYCIN, A NEW MEMBER OF PHOSPHOGLYCOLIPID ANTIBIOTICS. III. BIOLOGICAL PROPERTIES'
- FEMS MICROBIOL.LETT., Bd.23, Nr.2-3, Juli 1984 Seiten 289 - 291 A.D.RUSSELL 'Sensitivity of Escherichia coli and Pseudomonas aeruginosa to avoparcin, flavomycin and virginiamycin'

## Beschreibung

Die vorliegende Erfindung betrifft Moenomycin und dessen Derivate zur Herstellung von Arzneimitteln, sowie Arzneimittel mit einem Gehalt an Moenomycin oder dessen Derivaten.

Der vorliegenden Erfindung liegt die Aufgabe zugrunde, ein wirksames Medikament zur Bekämpfung von Magengeschwüren sowie zur Prophylaxe von Magenkrebs zu finden. Bisher wurden auf dem genannten Indikationsgebiet z.B. sogenannte Antacida und mit besonderem Erfolg H₂-Rezeptorenblocker benutzt.

Weiterhin wurde bereits erkannt, daß häufig Helicobacter pylori Infektionen für Magenerkrankungen verantwortlich sind. Die Infektion des menschlichen Magens mit dem pathogenen gram-negativen Bakterium Helicobacter pylori ruft ein vorübergehendes dyspeptisches Beschwerdebild hervor. H. pylori ist zudem der zugrundeliegende Erreger bei der chronisch-aktiven Typ-b-Gastritis und ein signifikanter Risikofaktor für das Auftreten von Magenkrebs. Die pathophysiologischen Mechanismen, durch welche H. pylori Krankheiten des Magens hervorruft, sind noch relativ unklar. Es ist bekannt, daß der Mikroorganismus eine Reihe von potentiell toxischen Enzymen und Chemikalien produziert (Urease, Ammoniak, vakuolisierendes Cytotoxin). Die Persistenz des Bakteriums und der dauernde antigene Stimulus sind wahrscheinlich die Ursache für die langfristige Zerstörung der Magenschleimhaut.

Das therapeutische Ziel ist die vollständige Eradikation von H. pylori. Therapie der Wahl ist momentan eine Dreifach-Kombination, die aus einem Wismutsalz, Metronidazol und Amoxicillin oder Tetracyclin besteht. Sie weist allerdings einige ernste Nebenwirkungen auf. Dazu zählen Mattigkeit, Mundtrockenheit, Durchfall und Übelkeit. Zudem muß der Patient während der Therapie auf Alkohol verzichten. Auch bei guter Compliance werden nur Eradikationsraten von ca. 90 % erreicht (A.T.R. Axon, 1993, J. Antimicrob. Chemother. 32, Suppl. A, 61 bis 68).

Überraschenderweise wurde nun gefunden, daß Moenomycin hervorragend gegen alle bisher untersuchten Helicobacter pylori Stämme wirksam ist. Dies ist insbesondere überraschend, weil bisher Moenomycin als nahezu ausschließlich gegen gram-positive Mikroorganismen wirksam bekannt war (Welzel et al., 1983, Tetrahedron Vol. 39, No. 9, 1583 bis 1591).

Erfindungsgegenstand ist demzufolge die Verwendung von Moenomycin und/oder einem oder mehreren seiner Derivate zur Herstellung eines Arzneimittels zur Bekämpfung von Magengeschwüren und zur Prophylaxe von Magenkrebs, sowie allgemein die Verwendung von Moenomycin und/oder einem oder mehreren seiner Derivate zur Herstellung eines Arzneimittels zur Bekämpfung von Helicobacter pylori Infektionen.

Das Moenomycin sowie viele seiner Derivate sind schon seit langem bekannt (vgl. DE-OS 3 704 659, EP 0 355 679, G. Huber in "Antibiotics", ed. F. Hahn, Springer Verlag, Berlin 1979, Vol. IV, Seite 135 ff., Welzel et al. in Tetrahedron a.a.O.). Moenomycine, z.B. das Moenomycin A, werden vorzugsweise durch Fermentation von Mikroorganismen und nachfolgende Aufreinigung gewonnen.

Unter dem Begriff Moenomycin im Sinne der vorliegenden Patentanmeldung ist ein Komplex von Moenomycin Komponenten (z.B. wie er von Mikroorganismen gebildet wird) sowie auch die einzelnen Komponenten zu verstehen. Das bedeutet, daß das Moenomycin in Form einer oder mehrerer Komponenten in variabler Zusammensetzung verabreicht werden kann. Bevorzugt ist die Verabreichung der weitgehend reinen besonders wirksamen Einzelkomponenten, insbesondere des Moenomycins A. Das genannte Moenomycin A hat die folgende Strukturformel:

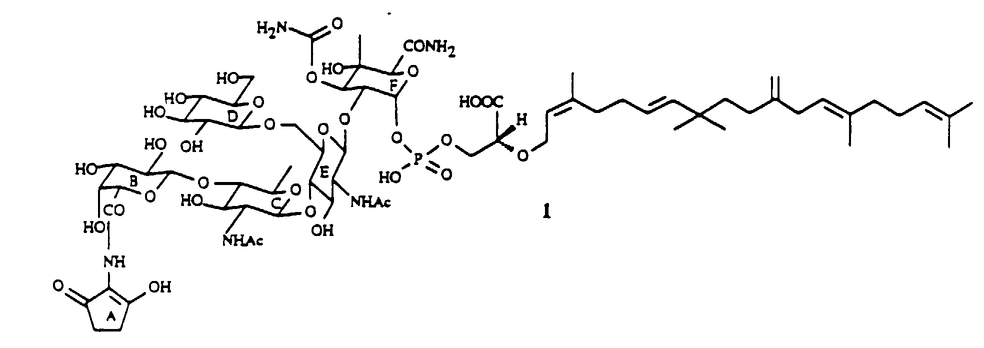

Mikroorganismen, die Moenomycin Komplexe produzieren sind z.B. Streptomyces bambergiensis, -ghanaensis, -ederensis und -geysirensis. Besonders bevorzugt ist Streptomyces bambergiensis (vgl. in diesem Zusammenhang Huber a.a.O.). Unter dem Begriff "Derivate des Moenomycins" sollen allgemein bisher synthetisierte Moenomycin Derivate verstanden werden. Besonders geeignete Moenomycin Komponenten bzw. Derivate des Moenomycins sind die Verbindungen der nachfolgenden Formeln. 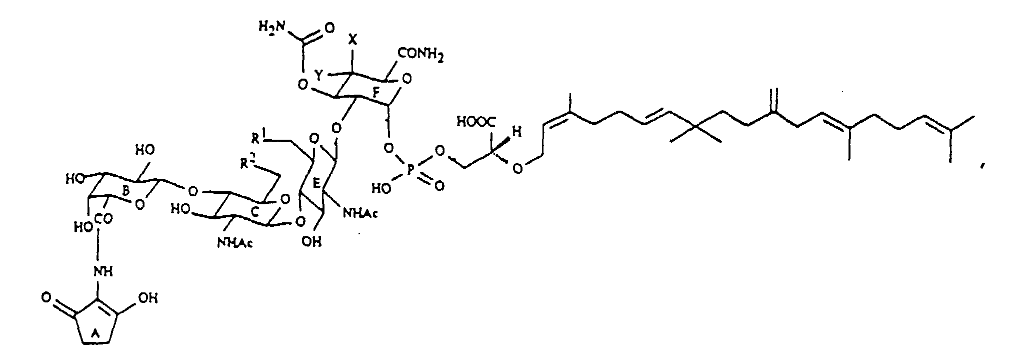 worin die einzelnen Substituenten die folgenden Bedeutungen haben,

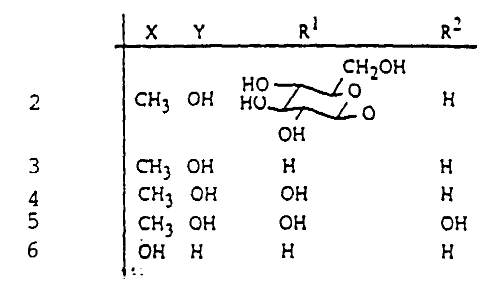

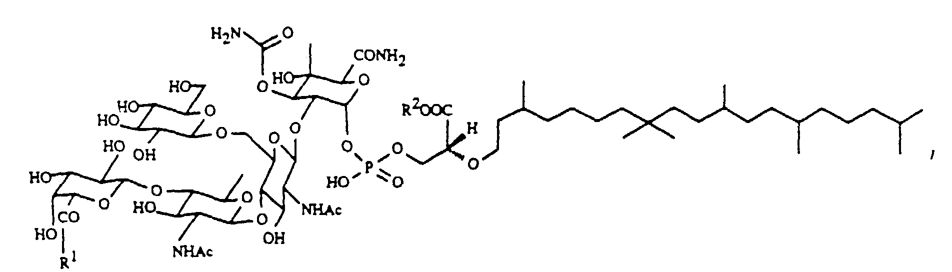

worin R₁ und R₂ die folgenden Bedeutungen haben,

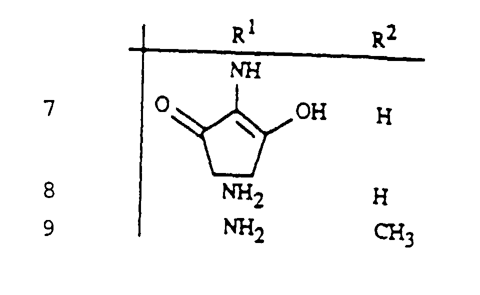

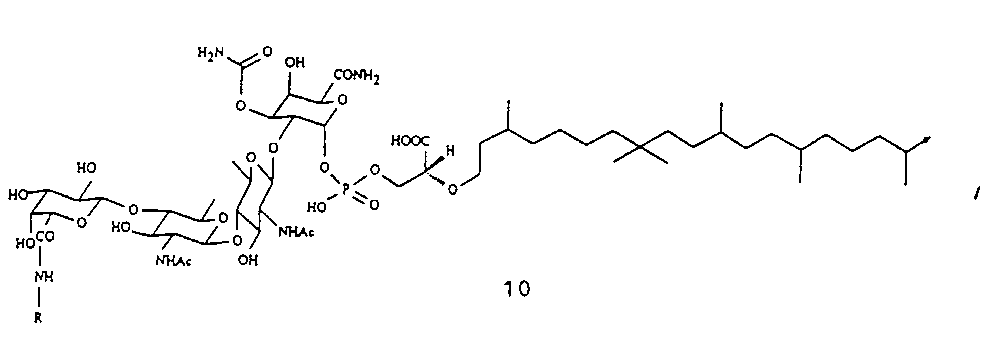

worin R Wasserstoff oder 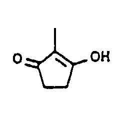 bedeutet,

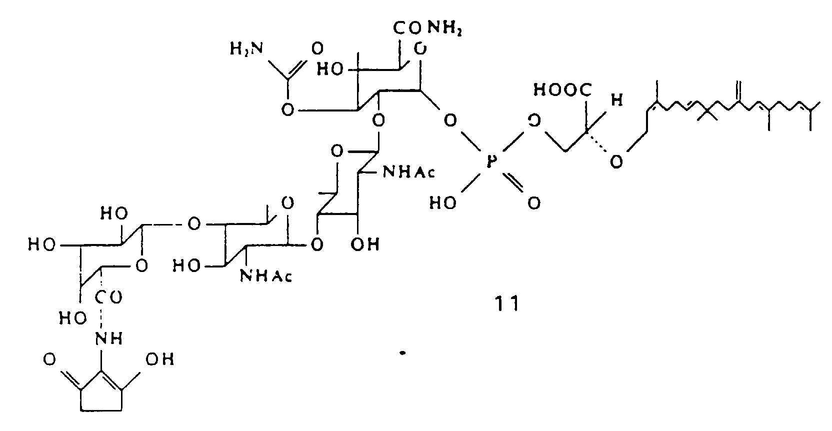

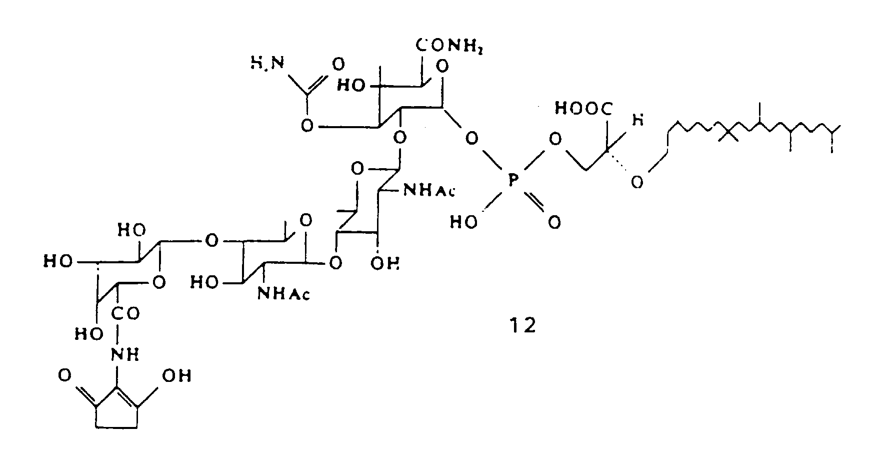

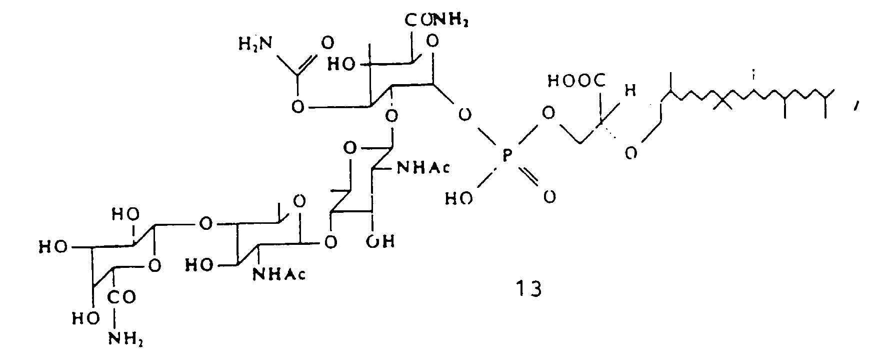

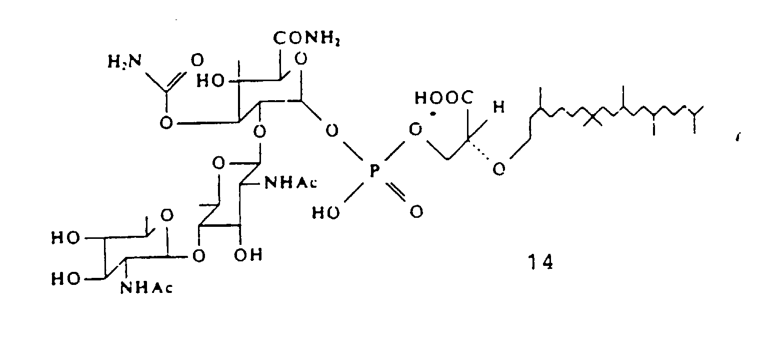

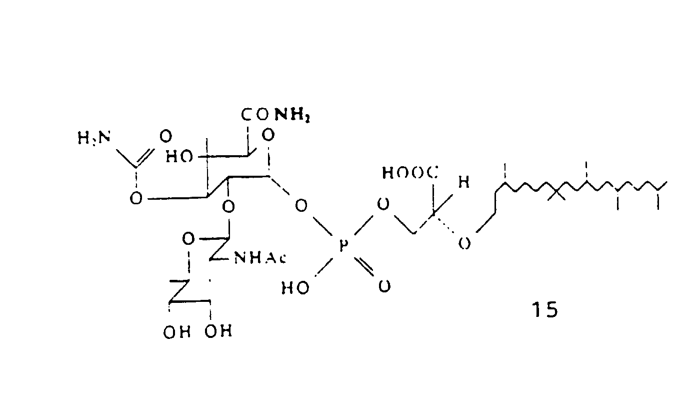

Die Verbindungen 11-15 repräsentieren die Abbauprodukte des Moenomycin C₃. In gleicher Weise sind analoge Abbauprodukte der Verbindungen 2, 4, 5 und 6 erfindungsgemäß einsetzbar.

Erfindungsgemäß besonders geeignet sind weiterhin Mischungen der genannten Verbindungen.

Die genannten Verbindungen lassen sich herstellen, wie z.B. beschrieben in G. Huber, a.a.O., DE-OS 37 04 659, Tetrahedron, Vol. 49, No. 35, pp. 7667-7678, 1993 und P. Welzel in "Antibiotics and Antiviral Compounds", VCH Weinheim, 1993.

Darüber hinaus eignen sich für die erfindungsgemäße Anwendung weitere Abbauprodukte von Moenomycin, wie die Abbauprodukte, die den vorstehenden Literaturstellen beschrieben sind, oder das Abbauprodukt der folgenden Formel, 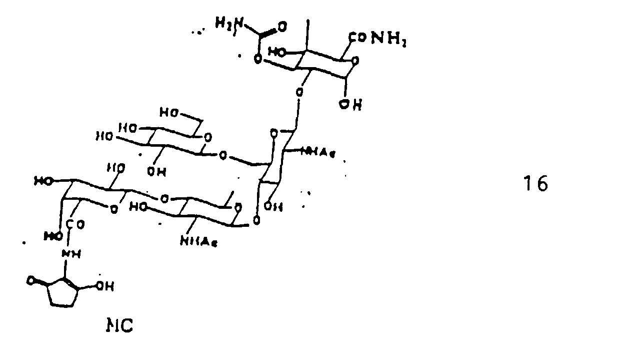 dessen Herstellung in der EP 0 355 679 beschrieben ist.

Die Anwendung von Moenomycin in der Therapie von H. pylori Infektionen weist gegenüber herkömmlicher Therapie eine Reihe von Vorteilen auf:

Das Antibiotikum wird nicht resorbiert und nahezu unverändert wieder ausgeschieden.

Moenomycin wird bisher in der Humanmedizin nicht angewendet. Das Problem von Kreuzresistenzen mit anderen Bakterienspezies entfällt.

Moenomycin ist extrem gut verträglich. Deshalb können hohe Dosierungen angewendet werden.

Moenomycin ist in der Lage, die Mucusschicht der Magenschleimhaut zu penetrieren und an den eigentlichen Aufenthaltsort des infizierenden Keimes zu gelangen.

Moenomycin besitzt keine Antigen- oder Hapteneigenschaften, die zu Allergien führen könnten.

Weitere Vorteile in der Therapie mit Moenomycin lassen sich erreichen, wenn man das Moenomycin bzw. dessen Derivate zusammen mit anderen Wirkstoffen, Hilfs- und/oder Trägerstoffen verabreicht.

Für die genannte Therapie geeignete zusätzliche Wirkstoffe stammen z.B. aus der Gruppe der Antacida, wie z.B. Natriumhydrogencarbonat, Aluminiumhydroxid, Magnesiumhydroxid, Magensiumtrisilikat, Aluminium-magnesium-silikathydrat, Aluminium-natrium-carbonat-dihydroxid, Magnesiumcarbonat, Calciumcarbonat oder Hydrotalcit.
Weitere geeignete zusätzliche Wirkstoffe stammen aus der Gruppe der H₂-Rezeptorenblocker wie z.B. Famotidin, Nizatidin, Roxatidinacetat, Ranitidin oder Cimetidin. Andere geeignete zusätzliche Wirkstoffe sind Muskarinrezeptorenblocker wie Propanthelinbromid, Pirenzipin oder andere Ulkusmittel wie Omeprazol, Lansoprazol, Misoprostol oder Wismutsalze wie Wismutnitrat, Wismutcarbont, Wismutsalicylat oder Wismutcitrat. Weitere für die Erfindungsgemäße Therapie geeignete zusätzliche Wirkstoffe gehören zur Gruppe der Antibiotika wie z.B. Tetracyclin, Metronidazol, Amoxycillin, Nisin, Clarithromycin, Imipenem oder Amikacin. Die vorstehend genannten zusätzlichen Wirkstoffe sind vorwiegend Handelsprodukte und nach allgemein bekannten Methoden erhältlich (vgl. Rote Liste 1993, Editio Cantor, Aulendorf, Württ., Merck Index, 11^{th} Ed., Merck & Co., Rahway, N.J., 1989).

Es kann auch sinnvoll sein, die Moenocmycin Therapie mit einer Mischung der obengenannten zusätzlichen Wirkstoffe durchzuführen.

Besonders bevorzugt ist die Verabreichung von Moenomycin zusammen mit Amoxycillin und/oder Metronidazol, Tetracyclin, Omeprazol, Ranitidin und/oder einem Wismutsalz.

Die Verabreichung der Komponenten der genannten Kombinationspräparate kann in Form einer einzigen Darreichung erfolgen oder auch in zeitlicher Abfolge vorgenommen werden.

Die galenische Zubereitung der erfindungsgemäßen Arzneimittel erfolgt nach Methoden des Standes der Technik z.B. in Form von Lösungen, Suspensionen, Kapseln, Tabletten, Rollkuren oder ähnlichem.

Die pharmazeutischen Zusammensetzungen, die den bzw. die Wirkstoffe enthalten, können in einer geeigneten Form sein für die orale Verabreichung, beispielsweise als Tabletten, Pastillen, Lutschbonbons, wässrige Suspensionen oder Lösungen, dispergierbare Pulver oder Granulate, Emulsionen, harte oder weiche Kapseln, Sirup oder Elixier. Für die orale Verabreichung bestimmte Zusammensetzungen können hergestellt werden nach jeder einschlägigen, dem Stand der Technik entsprechenden Methode für die Herstellung pharmazeutischer Zusammensetzungen, und diese Zusammensetzungen können eine oder mehrere Substanzen aus der Gruppe der Süßstoffe, Geschmacksstoffe, Farbstoffe und Konservierungsstoffe enthalten, um eine pharmazeutisch elegante und gut einzunehmende Zubereitung zu erhalten.

Formulierungen für die orale Verabreichung umfassen Tabletten die den Wirkstoff in einer Mischung mit nicht-toxischen, pharmazeutisch akzeptablen Trägerstoffen enthalten. Diese Trägerstoffe können beispielsweise inerte Streckmittel sein (wie etwa Natriumchlorid, Lactose, Kalciumphosphat oder Natriumphosphat), Granulations- oder Aufschlußmittel (zum Beispiel Kartoffelstärke, Alginsäure), Bindemittel (wie etwa Stärke, Gelatine oder Gummi Arabicum) und Schmiermittel (wie etwa Magnesiumstearat, Stearinsäure oder Talkum). Die Tabletten können unbeschichtet sein oder sie können mittels der bekannten Techniken beschichtet sein, um die Auflösung und Resorption im Magen zu verzögern und somit eine anhaltende Wirkung über einen längeren Zeitraum zu bieten. So kann beispielsweise ein zeitverzögernder Stoff wie etwa Glycerylmonostearat oder Glyceryldistearat eingesetzt werden.

Formulierungen für die orale Verabreichung können auch angeboten werden in Form von harten Gelatinekapseln, in denen der Wirkstoff mit einem inerten, festen Streckmittel, beispielsweise Calciumphosphat oder Kaolin, gemischt ist, oder in Form von weichen Gelatinekapseln, in denen der Wirkstoff mit Wasser oder einem öligen Medium, beispielsweise Erdnußöl, flüssiges Paraffin oder Olivenöl, gemischt ist.

Selbstverständlich variiert das therapeutische Dosierungsspektrum der erfindungsgemäßen Verbindungen je nach Größe und Bedürfnissen der Patienten und den jeweils zu behandelnden Schmerzen oder Krankheitssymptomen. Die Menge des Wirkstoffs, der mit Trägersubstanzen kombiniert werden kann, um eine einzige Darreichungsform zu bilden, variiert je nach dem zu behandelnden Wirt und der jeweiligen Verabreichungsart. So liegt beispielsweise für eine für Menschen bestimmte Formulierung für die orale Verabreichung vorzugsweise zwischen 5 mg und 5 g der jeweiligen Wirkstoff-Verbindung(en), wobei das Arzneimittel eine angemessene und sinnvolle Menge von Trägerstoffen enthalten sollte, die zwischen 5 und 95 % der Gesamtzusammensetzung ausmachen kann.
Es versteht sich, daß die spezifische Dosierung für jeden einzelnen Patienten von einer Vielzahl von Faktoren abhängig ist, einschließlich Wirksamkeit der spezifischen Verbindung, die eingesetzt wird, Alter, Körpergewicht, allgemeiner Gesundheitszustand, Geschlecht, Ernährung, Verabreichungszeit, Verabreichungsweg, Ausscheidungsgeschwindigkeit, Wechselwirkungen mit anderen Arzneimitteln und Schwere der jeweils behandelten Erkrankung.

Durch die nachfolgenden Ausführungsbeispiele soll die vorliegende Erfindung näher erläutert werden:

### Beispiel 1

Zur schnellen Bestimmung der Aktivität von Moenomycin werden definierte Mengen von Moenomycin in wässriger Lösung auf Filterplättchen getropft. Nach dem Trocknen werden die Plättchen auf Nähragarplatten gelegt, die mit Helicobacter pylori beimpft wurden.
Verschiedene klinische Isolate von Helicobacter pylori werden in flüssigem Nährmedium angezüchtet. Das Nährmedium besteht aus Müller-Hinton-Broth, dem 4 % fötales Kälberserum, 10 mg/l Vancomycin und 500 mg/l Actidione (die beiden letzteren zur Vermeidung von Kontaminationen) zugefügt sind. Das beimpfte Nährmedium wird zwei Tage lang unter speziellen Gasbedingungen (Anaerocult® C) bei 37°C gerührt. 100 µl der Bakteriensuspension werden auf der Oberfläche der Nähragarplatte ausgespatelt. Der Nähragar besteht aus Columbiaagar mit 5 % Hammelblut, dem wiederum 10 mg/l Vancomycin und 500 mg/l Actidion® zugesetzt sind. Nach dem Auflegen der Moenomycin enthaltenden Filterplättchen werden die Platten unter den gleichen Gasbedingungen wie die Flüssigkulturen erst 1 h bei 4°C und dann über 4 Tage bei 37°C bebrütet.

Typischerweise enthalten die Plättchen 25, 12,5, 6,25, 3,12 usw. µg Moenomycin.
Zur Auswertung wird der bakterienfreie Hemmhof ausgemessen, der ein Plättchen nach der Bebrütung umgibt. Der Durchmesser der Filterplättchen allein beträgt 6 mm.

Verwendet man Moenomycin A und den Bakerienstamm Helicobacter pylori P22, so erhält man folgendes Ergebnis:

| | | | | | | | | |
|---|---|---|---|---|---|---|---|---|
| [Moenomycin A] | 25 | 12,5 | 6,25 | 3,12 | 1,56 | 0,78 | 0,39 | 0,19 |
| Hemmhof (mm) | 34 | 32 | 30 | 28 | 23 | 20 | 16 | 11 |

### Beispiel 2

Verfährt man wie in Beispiel 1 beschrieben, setzt der Moenomycin Lösung vor dem Auftropfen auf die Filterplättchen jedoch ein handelsübliches Antazidum zu (z.B. Maalox® 70 in empfohlener Dosis), so erhält man bei Verwendung von Moenomycin A und des Helicobacter pylori Stammes P22 folgende Ergebnis:

| | | | | | | | | |
|---|---|---|---|---|---|---|---|---|
| [Moenomycin A] | 25 | 12,5 | 6,25 | 3,12 | 1,56 | 0,78 | 0,39 | 0,19 |
| Hemmhof (mm) | 32 | 30 | 25 | 25 | 25 | 25 | 24 | 23 |

Das Ergebnis läßt auf eine Wirksamkeitssteigerung von Moenomycin A durch ein Antazidum im unteren Konzentrationsbereich schließen. Verwendet man andere Helicobacter pylori Stämme, so erhält man ähnlich verlaufende Kurven.

### Beispiel 3

### Bestimmung der minimalen Hemmkonzentration von Moenomycin A

Zur Bestimmung der minimalen Hemmkonzentration von Moenomycin A gegen verschiedene Helicobacter pylori Stämme in vitro wurde der Agar - Verdünnungstest verwendet, der dem Fachmann bekannt ist. Dazu wurden verschiedene Helicobacter pylori Stämme in Flüssigkultur angezüchtet, wie in Beispiel 1 beschrieben. In Agarplatten wurden Verdünnungen von Moenomycin A eingegossen, so daß bestimmte Konzentrationen des Antibiotikums eingestellt wurden (typischerweise 100, 50, 25, 12,5 usw µg/ml). Mit Hilfe eines Vielpunkt - Beimpfungsgerätes wurden die gleichen Stämme von Helicobacter pylori auf jede Platte aufsteigender Konzentration aufgeimpft und wie in Beispiel 1 bebrütet. Der Konzentrationspunkt, an welchem gerade kein Wachstum mehr feststellbar war, wurde als der MHK-Wert (minimale Hemmkonzentration) für den entsprechenden Stamm abgelesen. Verwendet man beispielsweise die Helicobacter pylori Stämme P9, P19 und M84, so liegt für sie der MHK-Wert zwischen 0,19 und 0,09 µg/ml.

## Patentansprüche

1. Verwendung von Moenomycin und/oder einem oder mehreren seiner Derivate zur Herstellung eines Arzneimittels zur Bekämpfung von Magengeschwüren und zur Prophylaxe von Magenkrebs.

2. Verwendung von Moenomycin und/oder einem oder mehreren seiner Derivate zur Herstellung eines Arzneimittels zur Bekämpfung von Helicobacter pylori Infektionen.

3. Arzneimittel, dadurch gekennzeichnet, daß sie neben Moenomycin und/oder einem oder mehreren seiner Derivate mindestens ein weiteres Mittel gegen Magengeschwüre und/oder ein Antibiotikum enthalten.

4. Verfahren zur Herstellung eines Arzneimittels gemäß Anspruch 3, dadurch gekennzeichnet, daß Moenomycin und/oder eines oder mehrere seiner Derivate mit mindestens einem weiteren Mittel gegen Magengeschwüre und/oder einem Antibiotikum ggf. mit pharmakologisch verträglichen Hilfs- und/oder Trägerstoffen in eine geeignete Darreichungsform gebracht werden.

## Claims

1. The use of moenomycin and/or one or more of its derivatives for the production of a pharmaceutical for the control of gastric ulcers and for the prophylaxis of cancer of the stomach.

2. The use of moenomycin and/or one or more of its derivatives for the production of a pharmaceutical for the control of Helicobacter pylori infections.

3. A pharmaceutical, which in addition to moenomycin and/or one or more of its derivatives contains at least one further agent against gastric ulcers and/or an antibiotic.

4. A process for the production of a pharmaceutical as claimed in claim 3, which comprises bringing moenomycin and/or one or more of its derivatives into a suitable form for administration with at least one further agent against gastric ulcers and/or an antibiotic, if appropriate using pharmacologically tolerable auxiliaries and/or excipients.

## Revendications

1. Utilisation de la moénomycine et/ou d'un ou plusieurs de ses dérivés, pour la fabrication d'un médicament destiné à la lutte contre des ulcères gastriques et à la prophylaxie du cancer de l'estomac.

2. Utilisation de la moénomycine et/ou d'un ou plusieurs de ses dérivés, pour la fabrication d'un médicament destiné à la lutte contre des infections à *Helicobacter pylori.*

3. Médicaments, caractérisés en ce qu'ils contiennent, outre de la moénomycine et/ou un ou plusieurs de ses dérivés, au moins un autre agent contre les ulcères gastriques et/ou un antibiotique.

4. Procédé pour la fabrication d'un médicament selon la revendication 3, caractérisé en ce que l'on met sous une forme d'administration appropriée de la moénomycine et/ou un ou plusieurs de ses dérivés, avec au moins un autre agent contre les ulcères gastriques et/ou un antibiotique, et éventuellement avec des adjuvants et/ou véhicules pharmacologiquement acceptables.
